# EUROPÄISCHE PATENTSCHRIFT

(11) **EP 2 468 316 B1**
(45) Veröffentlichungstag und Bekanntmachung des Hinweises auf die Patenterteilung: **07.09.2016**
(21) Anmeldenummer: 11009582.5
(22) Anmeldetag: 05.12.2011
(51) Int. Cl.: A61L 27/54, A61L 31/16, B05C 1/06, B05D 1/28

(54) **Beschichtungsvorrichtung**
Surface coating device
Dispositif de revêtement

(30) Priorität: 23.12.2010 DE 102010055562
(43) Veröffentlichungstag der Anmeldung: 27.06.2012
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 90491 Nürnberg (DE)
(74) Vertreter: Heraeus IP

(56) Entgegenhaltungen:
- WO-A1-2005/042045
- WO-A2-2008/086794
- US-A1- 2008 260 936
- US-B1- 7 563 324

## Beschreibung

Die Erfindung betrifft eine Vorrichtung zum Beschichten eines medizinischen Implantats.

Die Beschichtung von medizinischen Implantaten mit pharmazeutischen Wirkstoffen hat in den letzten Jahren zunehmend Beachtung gefunden. Eine zentrale Anwendung von Beschichtungsverfahren liegt dabei im antibiotischen Schutz der Oberfläche von Implantatmaterialien. Eine weitere wichtige Anwendung ist in der Verbesserung der Oberflächenkompatibilität von nicht zu zementierenden medizinischen Implantaten zum besseren Anwachsen von Knochenmaterial zu sehen.

Die Implantation von Gelenkendoprothesen und auch von Osteosynthesematerialien ist immer mit einer gewissen Gefahr einer mikrobiellen Kontamination verbunden. Wenn es mikrobiellen Keimen gelingt, sich an der Implantatoberfläche anzusiedeln, kann es zur Ausbildung einer post-operativen Osteitis/Osteomyelitis kommen. Die Osteitis/Osteomyelitis stellt eine schwerwiegende Komplikation für den Patienten dar, die zusätzlich mit erheblichen Kosten verbunden ist.

Seit Jahrzehenten wird mit klinischem Erfolg bei zementierten Gelenkendoprothesen mit Gentamicin dotierter PMMA-Knochenzement verwendet. Das im Knochenzement enthaltende Breitbandantibiotikum Gentamicin schützt die Oberfläche des Knochenzementes wirksam gegen bakterielle Infektionen.

In WO2005/042045 A1 sind ein Verfahren und eine Vorrichtung zum Auftragen einer definierten Menge eines Beschichtungsmaterials auf die Oberfläche eines Implantates mittels eines Druckverfahrens beschrieben. Die Beschichtung wird mit Hilfe einer Druckwalze aufgetragen.

US 7,563,324 B1 beschreibt eine Vorrichtung und ein Verfahren zur Beschichtung einer implantierbaren medizinischen Einrichtung, insbesondere eines Stents. Die Vorrichtung enthält ein Reservoir für die Beschichtungzusammensetzung und einen Applikator zum Auftragen der Beschichtungszusammensetzung. Über einen porösen Bereich wird die Beschichtungszusammensetzung aus dem Reservoir auf den Applikator übertragen.

WO 2008/086794 A2 betrifft ein Verfahren zur Beschichtung von Katheterballons mit einer definierten Menge eines pharmakologischen Wirkstoffs, wobei das Beschichtungsverfahren eine Beschichtungsvorrichtung verwendet, welche mit einer Volumenmesseinrichtung zur Abgabe einer messbaren Menge einer Beschichtungslösung mittels einer Abgabevorrichtung gezielt auf die Oberfläche des Katheterballons versehen ist.

US 2008/0260936 A1 beschreibt Verfahren zur selektiven Beschichtung von medizinischen Vorrichtungen, wie expandierbaren Stents, wobei eine Beschichtung auf die medizinische Vorrictung aufgebracht wird und anschließend über die gesamte zugängliche Fläche verteilt wird.

Bei nicht-zementierten Gelenkendoprothesen und bei Osteosynthesematerialien wurde eine Reihe von Lösungswegen vorgeschlagen, um ebenfalls einen lokalen antibiotischen Schutz der Implantatoberflächen zu erreichen.

So wurde in mehreren Patentschriften die Verwendung von in Wasser gering löslichen Antibiotika-Salzen beschrieben. Exemplarisch seien dafür die EP 0 623 349 A1, EP 1 470 829 A1, EP 1 374 923 A2, DE 101 42 465 A1 und DE 44 04 018 A1 genannt. Diese in Wasser gering löslichen Salze lösen sich unter Freisetzung der enthaltenen Antibiotika durch Einwirkung von Körperflüssigkeiten auf. Vorteilhaft ist die protrahierte Wirkstofffreisetzung. Nachteilig ist jedoch die aufwendige Herstellung dieser Salze.

Alternativ dazu ist es auch möglich, in Wasser lösliche Antibiotika-Salze zu verwenden. Das Problem liegt dabei darin, das Antibiotikum auf der Implantatoberfläche zu fixieren.

Die Mehrzahl der bisherigen beschriebenen Beschichtungen ist vorzugsweise für die Fertigung von beschichteten Implantaten unter industriellen Bedingungen bestimmt. Das bedeutet, dass die industrielle Beschichtung dieser Implantate nur mit wenigen für die Großanwendung relevanten Wirkstoffen erfolgen kann, um eine wirtschaftliche industrielle Fertigung mit entsprechenden hohen Stückzahlen gewährleisten zu können.

Insbesondere bei antibiotischen Beschichtungen ist es jedoch im Hinblick auf die sich zunehmend problematisch gestaltende Resistenzsituation und dem dabei vermehrten Auftreten von multiresistenten Keimen, wie MRSA und MRSE von Interesse, genau auf die jeweiligen Keime abgestimmte Antibiotika oder Antibiotika-Kombinationen zur Beschichtung von Revisionsprothesen im Zuge des einzeitigen oder zweizeitigen septischen Gelenkprothesenwechsels einzusetzen, um einen initialen antibiotischen Schutz der Implantatoberflächen zu gewährleisten.

Nachteilig ist hieran des Weiteren, dass die Verfahren zur Beschichtung der medizinischen Implantate relativ aufwendig sind. Eine variable kurzfristige Anwendung ist nicht möglich. Es müssen für verschiedene Anforderungen verschiedene beschichtete medizinische Implantate bereitgehalten werden, um den Bedürfnissen der unterschiedlichen Patienten gerecht zu werden. Dies erfordert eine umfangreiche Lagerhaltung und verhindert ungewöhnliche Mischungen für spezielle Fälle.

Die Aufgabe der Erfindung besteht darin, die Nachteile des Stands der Technik zu überwinden. Insbesondere soll eine einfache und leicht anzuwendende Vorrichtung bereitgestellt werden, mit der eine Oberfläche eines medizinischen Implantats, wie einer Prothese, beschichtet werden kann, ohne dass dadurch der Ablauf einer Operation (OP) gestört wird. Es sollen möglichst viele verschiedene medizinische Implantate mit der gleichen Vorrichtung beschichtet werden können. Auch soll die Vorrichtung variabel einsetzbar sein, so dass sie an die medizinischen Notwendigkeiten, insbesondere an eine für den Patienten geeignete Medikamentierung, anpassbar sind. Der in Operationssälen gebotenen Reinheit soll ebenfalls Rechnung getragen werden.

Die Aufgabe der Erfindung besteht ferner darin, eine möglichst einfache Beschichtungsvorrichtung und ein möglichst einfaches Beschichtungsverfahren zu entwickeln, das vom OP-Personal während einer Operation mit geringstem Zeitaufwand zum Beschichten von unterschiedlichsten Implantaten, die von beliebigen Herstellern produziert sein können, mit pharmazeutischen Zubereitungen eingesetzt werden kann. Dabei soll es zudem möglich sein, unter OP-Bedingungen Implantate beliebiger Hersteller mit geringem Aufwand unter Verwendung beliebiger flüssiger pharmazeutischer Zubereitungen zu beschichten. Auch soll die Vorrichtung selbst keine Kunststoffpartikel, Metallpartikel oder andere nicht oder nur teilweise biodegradierbare Partikel freisetzen. Eine weitere Aufgabe besteht darin, dass die Vorrichtung besonders zur Beschichtung von nicht-zementierten Gelenkendoprothesen und Osteosynthesematerialien geeignet sein soll. Zusätzlich soll ein möglichst einfaches Beschichtungsverfahren entwickelt werden.

Die Aufgabe der Erfindung wird dadurch gelöst, dass die Vorrichtung einen Behälter umfasst, der eine Flüssigkeit beinhaltet, wobei die Flüssigkeit zumindest eine pharmazeutisch aktive Substanz umfasst, die Vorrichtung einen elastischen Kopf zum Auftragen der Flüssigkeit auf einer zu beschichtenden Oberfläche umfasst, der über zumindest einen Kanal mit dem Inneren des Behälters verbunden ist, so dass bei einem Überstreichen oder Überrollen der zu beschichtenden Oberfläche mit dem elastischen Kopf die Flüssigkeit die die zu beschichtende Oberfläche benetzt.

Unter einer pharmazeutisch aktiven Substanz sind erfindungsgemäß pharmazeutisch wirksame Mittel und Mittel zu verstehen, die pharmakologisch wirken, sowie solche, die eine pharmakologische Wirkung unterstützen oder sonst in irgendeiner Weise die Selbstheilungskräfte des Körpers unterstützen. Beispiele sind hierfür Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Unter einer Flüssigkeit ist erfindungsgemäß auch eine zähflüssige Flüssigkeit zu verstehen. Die Breite oder die Querschnitte der Kanäle oder des Kanals können dazu an die Viskosität der Flüssigkeit angepasst sein.
besondere an eine für den Patienten geeignete Medikamentierung, anpassbar ist. Der in Operationssälen gebotenen Reinheit soll ebenfalls Rechnung getragen werden.

Die Aufgabe der Erfindung besteht ferner darin, eine möglichst einfache Beschichtungsvorrichtung zu entwickeln, die vom OP-Personal während einer Operation mit geringstem Zeitaufwand zum Beschichten von unterschiedlichsten Implantaten, die von beliebigen Herstellern produziert sein können, mit pharmazeutischen Zubereitungen eingesetzt werden kann. Dabei soll es zudem möglich sein, unter OP-Bedingungen Implantate beliebiger Hersteller mit geringem Aufwand unter Verwendung beliebiger flüssiger pharmazeutischer Zubereitungen zu beschichten. Auch soll die Vorrichtung selbst keine Kunststoffpartikel, Metallpartikel oder andere nicht oder nur teilweise biodegradierbare Partikel freisetzen. Eine weitere Aufgabe besteht darin, dass die Vorrichtung besonders zur Beschichtung von nicht-zementierten Gelenkendoprothesen und Osteosynthesematerialien geeignet sein soll. Zusätzlich soll ein möglichst einfaches Beschichtungsverfahren entwickelt werden.

Die Aufgabe der Erfindung wird dadurch gelöst, dass die Vorrichtung einen Behälter umfasst, der eine Flüssigkeit enthält, die zumindest eine pharmazeutisch aktive Substanz umfasst, wobei die Vorrichtung einen elastischen Kopf zum Auftragen der Flüssigkeit auf eine zu beschichtende Oberfläche umfasst, der über zumindest einen Kanal mit dem Inneren des Behälters verbunden ist. Vorzugsweise wird so bei einem Kontaktieren, insbesondere bei einem Überstreichen oder Überrollen, der zu beschichtenden Oberfläche mit dem elastischen Kopf die zu beschichtende Oberfläche mit der Flüssigkeit benetzt, wobei der Boden und/oder die Wand des Behälters zumindest bereichsweise eine elastische durchstechbare Membran umfasst.

Unter einer pharmazeutisch aktiven Substanz sind erfindungsgemäß pharmazeutisch wirksame Mittel und Mittel zu verstehen, die pharmakologisch wirken, sowie solche, die eine pharmakologische Wirkung unterstützen oder sonst in irgendeiner Weise die Selbstheilungskräfte des Körpers unterstützen. Beispiele sind hierfür Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Unter einer Flüssigkeit ist erfindungsgemäß auch eine zähflüssige Flüssigkeit zu verstehen. Die Breite oder die Querschnitte der Kanäle oder des Kanals können dazu an die Viskosität der Flüssigkeit angepasst sein.

Gemäß einer besonders vorteilhaften Ausgestaltung der Erfindung kann vorgesehen sein, dass der Behälter elastische Wände hat, so dass bei einem manuellen Druck auf die elastischen Behälterwände die Flüssigkeit durch den zumindest einen Kanal zur Oberfläche des elastischen Kopfs strömt. Hierdurch wird erreicht, dass die Menge der austretenden Flüssigkeit durch manuellen Druck gesteuert werden kann.

Erfindungsgemäße Verfahren können sich auch dadurch auszeichnen, dass der elastische Kopf eine rotationssymmetrische Walze ist, die um ihre Symmetrieachse drehbar an einem Ende des Behälters gelagert ist.

Eine weitere erfindungsgemäße Ausgestaltung des Verfahrens kann vorsehen, dass der elastische Kopf eine Kugel ist, die um zumindest eine Achse drehbar an einem Ende des Behälters gelagert ist. Die Drehbarkeit des elastischen Kopfs hat den besonderen Vorteil, dass die Flüssigkeit im Wesentlichen ohne eine Gleitreibung auf das medizinische Implantat aufgetragen werden kann. Dadurch wird zu einen der Abrieb des elastischen Kopfs und damit störende Partikel am medizinischen Implantat vermieden, zum anderen ist der Übertrag der Flüssigkeit auf das medizinische Implantat durch diese Maßnahme besonders gut zu steuern. Dabei kann vorgesehen sein, dass die Kugel frei drehbar an einem Ende des Behälters gelagert ist.

Ferner kann vorgesehen sein, dass zumindest ein Kanal zwischen der Behälterwand und dem elastischen Kopf gebildet ist. Dies ist insbesondere im Zusammenhang mit einem drehbar gelagerten elastischen Kopf vorteilhaft, da durch die Drehung des elastischen Kopfs die Flüssigkeit, aus dem Behälter an die Oberfläche der Vorrichtung transportiert wird, und dann auf die Oberfläche des medizinischen Implantats übertragbar ist.

Auch kann vorgesehen sein, dass ein zur Behandlungssituation passendes Antibiotikum oder Antibiotika-Gemisch in der Flüssigkeit in den Behälter einfüllbar ist. Durch diese Maßnahme kann auf die konkrete Behandlungssituation beim jeweiligen Patienten eingegangen werden.

Gemäß einer besonders bevorzugten Ausgestaltung der Erfindung kann vorgesehen sein, dass die pharmazeutisch aktive Substanz Antibiotika und/oder organische Antiseptika beinhaltet, so dass die zu erzeugende Beschichtung eine pharmazeutisch wirksame Dosis beinhaltet.

Es kann auch vorgesehen sein, dass der elastische Kopf einen porösen elastischen Schwamm umfasst. Der poröse elastische Schwamm kann erfindungsgemäß dazu genutzt werden, dass die Flüssigkeit in einem gleichmäßigen Film auf dem medizinischen Implantat aufgetragen wird.

Eine weitere Ausgestaltung der Erfindung schlägt vor, dass der elastische Kopf linear verschiebbar im Behälter angeordnet ist. Insbesondere kann vorgesehen sein, dass der elastische Kopf in den Behälter hineindrückbar ist. Dadurch wird die Flüssigkeit aus dem Inneren des Behälters nach außen gedrückt, wenn der elastische Kopf auf eine Oberfläche gedrückt wird.

Es kann auch vorgesehen sein, dass ein Kanal im elastischen Kopf ausgebildet ist, vorzugsweise durch Poren eines Schwamms.

Ferner kann vorgesehen sein, dass am elastischen Kopf ein unelastischer Pfropfen angeordnet ist, der den Behälter einseitig abschließt und der zumindest einen Kanal umfasst und/oder zumindest einen Kanal zwischen dem Pfropfen und dem Behälter bildet, wobei der Pfropfen vorzugsweise linear verschiebbar im Behälter angeordnet ist. Dies stellt die Stabilität der Vorrichtung sicher.

Gemäß einer weiteren Ausgestaltung der Erfindung ist vorgesehen, dass der elastische Kopf eine raue Oberfläche hat, vorzugsweise eine Rauigkeit von 0,5 µm bis 100 µm hat, besonders bevorzugt 1 µm bis 10 µm, ganz besonders bevorzugt 2 µm. Dies unterstützt, insbesondere bei einem drehbar gelagerten elastischen Kopf, die stabile Förderung der Flüssigkeit an die Oberfläche der Vorrichtung und damit auf das medizinische Implantat.

Zu diesem Zweck kann ferner vorgesehen sein, dass der elastische Kopf hydrophil ist und die Flüssigkeit eine wässrige Lösung ist.

Um den besonderen hygienischen Anforderungen zu entsprechen kann vorgesehen sein, dass der elastische Kopf den Behälter bis auf die Verbindung durch den zumindest einen Kanal nach außen abschließt.

Um eine einfache Anwendbarkeit und Befüllbarkeit des Behälters zu gewährleisten, ist vorgesehen, dass der Boden und/oder zumindest eine Wand des Behälters zumindest bereichsweise eine elastische durchstechbare Membran umfasst, wobei die Membran vorzugsweise aus einem biokompatiblen Elastomer gebildet ist und/oder durch einen Deckel abdeckbar und/oder abdichtbar ist.

Auch kann vorgesehen sein, dass die Vorrichtung eine Lagerung für den elastischen Kopf umfasst, die mit dem Behälter verbunden ist oder mit dem Behälter in einem ausgeformt ist.

Besonders vorteilhafte Ausgestaltungen der Erfindung sehen vor, dass der elastische Kopf einen Elastizitätsmodul von weniger als 2000 MPa, bevorzugt von weniger als 500 MPa, besonders bevorzugt zwischen 1 und 100 MPa hat. Bei diesen Elastizitätsmoduln ist ein flächiges Auftragen der Flüssigkeit auch bei unebenen zu beschichtenden Oberflächen des medizinischen Implantats möglich, wenn die erfindungsgemäße Vorrichtung mit der Hand bedient wird.

Auch kann vorgesehen sein, dass zumindest ein Kanal, bevorzugt alle Kanäle, einen Querschnitt von unter 500 µm hat, vorzugsweise mit einem Querschnitt von unter 200 µm.

Ferner kann vorgesehen sein, dass zumindest ein Kanal ein Spalt mit einer Breite von unter 500 µm ist, vorzugsweise mit einer Breite von unter 200 µm, bevorzugt alle Kanäle solche Spalten sind. Bei diesen Kanalquerschnitten beziehungsweise Kanalbreiten wird für den medizinischen Zweck ein besonders gut geeigneter Flüssigkeitsfilm aufgetragen.

Erfindungsgemäß kann auch vorgesehen sein, dass der elastische Kopf und/oder der Behälter ein biokompatibles Material umfasst oder umfassen, vorzugsweise zumindest die Oberfläche des elastischen Kopfs aus einem biokompatiblen Elastomer oder Elastomergemisch besteht.

Auch kann vorgesehen sein, dass der elastische Kopf und/oder der Behälter aus einem biokompatiblen Material gefertigt ist oder sind.

Eine besonders gut handhabbare Ausgestaltung der erfindungsgemäßen Vorrichtung ergibt sich, wenn vorgesehen ist, dass das durch den Behälter und den elastischen Kopf eingeschlossene Volumen zwischen 0,5 ml und 1000 ml, vorzugsweise zwischen 1 ml und 100 ml beträgt.

Auch kann vorgesehen sein, dass der Behälter sterile Luft oder ein steriles Gas beinhaltet.

Ferner kann vorgesehen sein, dass zwischen dem elastischen Kopf und dem Behälter wenigstens eine offenporige poröse Schicht angeordnet ist, vorzugsweise im Inneren der Vorrichtung. Die offenporige poröse Schicht soll sicherstellen, dass immer genug Flüssigkeit im Bereich des elastischen Kopf vorhanden ist.

Eine weitere Ausgestaltung der Erfindung sieht vor, dass die Flüssigkeit eine wässrige Gentamicinsulfat-Lösung umfasst, vorzugsweise umfassend Stabilisatoren, wobei besonders eine wässrige Gentamicinsulfat-Lösung mit einem Gentamicinsulfat-Gehalt von 0,5 bis 88 Gewichtsprozent bevorzugt ist.

Dabei kann vorgesehen sein, dass die Flüssigkeit eine Gentamicinsulfat-Lösung mit einem Gentamicinsulfat-Gehalt von 10 bis 88 Gewichtsprozent ist, besonders bevorzugt eine Gentamicinsulfat-Lösung mit einem Gentamicinsulfat-Gehalt von 80 Gewichtsprozent ist.

Nach einem Verfahren zum Beschichten eines medizinischen Implantats mit einer erfindungsgemäßen Vorrichtungwird ein medizinisches Implantat bereitgestellt und der elastische Kopf der Vorrichtung mit der zu beschichtenden Oberfläche des medizinischen Implantats kontaktiert, vorzugsweise über die zu beschichtende Oberfläche des medizinischen Implantats gerollt oder gestrichen, so dass die Flüssigkeit als Film von dem elastischen Kopf auf die Oberfläche des medizinischen Implantats übertragen wird.

Dabei ist vorgesehen, dass der Behälter der Vorrichtung durch Befüllen mit einer Flüssigkeit, vorzugsweise durch Einspritzen der Flüssigkeit durch die Membran, mit der Flüssigkeit gefüllt wird. Durch diese Maßnahme kann auf die konkrete Behandlungssituation beim jeweiligen Patienten eingegangen werden.

Dabei kann wiederum vorgesehen sein, dass ein Deckel abgenommen wird, bevor der Behälter mit der Flüssigkeit gefüllt wird und/oder ein Deckel, vorzugweise über einer Einstichstelle befestigt wird, nachdem der Behälter mit der Flüssigkeit gefüllt wurde. Mit der Verwendung eines Deckels soll ein versehentliches Öffnen oder Durchstechen der Behälterwand verhindert werden und der Behälter, nachdem die Behälterwand beziehungsweise der Boden des Behälters durchstochen oder geöffnet wurde, wieder abgedichtet werden, damit keine Verunreinigungen in den Behälter gelangen können und der Inhalt der Vorrichtung die Umgebung, das heißt den OP-Bereich, nicht kontaminieren kann.

Die Verfahren erfolgen vor dem Einsetzen der medizinischen Implantate. Die Verfahren finden also "ex vivo" statt.

Ferner kann vorgesehen sein, dass zumindest 50% der Oberfläche des medizinischen Implantats, vorzugsweise zumindest 80%, besonders bevorzugt zumindest 90% der Oberfläche des medizinischen Implantats beschichtet werden.

Es kann ferner vorgesehen sein, dass das Verfahren so oft wiederholt wird, bis eine vollständige Beschichtung der zu beschichtenden Oberfläche des medizinischen Implantats erreicht wird. Insbesondere im Zusammenhang mit einer Färbung der Flüssigkeit und der Prüfung der Vollständigkeit der Beschichtung mit Hilfe der Färbung ist dies erfindungsgemäß vorteilhaft, um eine ausreichend beschichtetes medizinisches Implantat zu erzeugen.

Die Vorrichtung kann erfindungsgemäß mit einer Lösung oder Suspension umfassend die zumindest eine pharmazeutisch aktive Substanz vorbefüllt sein, so dass das OP-Personal sofort

Alternativ ist es möglich, eine nicht befüllte Vorrichtung direkt im OP durch Einspritzen einer Wirkstofflösung oder Wirkstoffsuspension mit einem oder mehreren pharmazeutischen Wirkstoffen auszurüsten. Dadurch ist es im Fall der antibiotischen Beschichtung möglich, entsprechend der vorliegenden Resistenzsituation eine geeignete Auswahl eines Antibiotikums oder einer Antibiotika-Kombination vorzunehmen und damit eine Antibiogramm-gerechte Beschichtung herzustellen.

Es ist auch möglich, vor der OP nicht befüllte Vorrichtungen in der jeweiligen Klinikapotheke mit geeigneten Wirkstofflösungen oder Wirkstoffsuspensionen zu befüllen, so dass während der OP die Beschichtung ohne Zeitverzug vorgenommen werden kann.

Beispiele für verwendbare pharmazeutische Wirkstoffe sind Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Im Rahmen der Erfindung ist ebenfalls die Verwendung von anderen Aminoglykosid-AntibiotikaLösungen, wie wässrige Lösungen des Tobramycinsulfats, des Amikacinsulfats, des Netilmicinsulfats und des Sisomycinsulfats. Es ist auch möglich, wässrige Lösungen des Vancomycins, des Dalbavancins, des Ramoplanins, des Daptomycins, des Moxifloxacins, des Clindamycins und/oder des Lincomycins einzusetzen. Ebenfalls im Rahmen der Erfindung ist die Verwendung von Kombinationen von Lösungen verschiedener Antibiotika. Beispielhaft sind hierbei die Zweifachkombinationen von Gentamicinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid. Weiterhin ist es möglich, anstelle von Antibiotika-Lösungen auch Antiseptika-Lösungen zu verwenden. Beispielhaft sind hierbei Lösungen des Chlorhexidindigluconats, des Octenidindihydrochlorids und des Polyhexanids.

Ebenfalls im Rahmen der Erfindung ist die Verwendung von Lösungen von Antibiotika und Antiseptika, die als Lösungsmittel organische Lösungsmittel oder Kombinationen von organischen Lösungsmitteln oder auch Kombinationen von organischen Lösungsmitteln und Wasser enthalten.

Es ist dadurch möglich, zum Beispiel auch in Wasser gering lösliche Antibiotika-Salze, wie Laurate, Myristate, Palmitate und Stearate zu verwenden. Daneben können auch in Wasser gering lösliche Antibiotika oder Antibiotika-Salze in Form von wässrigen Suspensionen verwendet werden.

Erfindungsgemäß ist es ferner, dass die Vorrichtung als Arzneimittel oder als Medizinprodukt bereitgestellt wird.

Auch eine Kombination der erfindungsgemäßen Vorrichtung mit einem medizinischen Implantat könnte angeboten werden. Diese Kombination wird aus der Vorrichtung und dem Implantat gebildet, wobei diese Kombination eine Mindestlebensdauer von 0,1 Sekunden hat. Die Kombination entsteht besteht während des Beschichtungsprozesses.

Der Erfindung liegt die überraschende Erkenntnis zugrunde, dass eine Flüssigkeit, mit der ein medizinisches Implantat beschichtet werden soll, auch kurz vor seiner Anwendung durch Kontaktieren, vorzugsweise durch Überstreichen des medizinischen Implantats mit einer erfindungsgemäßen Vorrichtung, aufgetragen werden kann, in der die Flüssigkeit gespeichert ist. Das einfach gestaltete Verfahren und die Vorrichtung hierfür stellen dabei die Anwendbarkeit auch im OP-Bereich sicher. Die aufzutragende Flüssigkeit, die die pharmazeutisch aktive Substanz beinhaltet, wird dazu durch einen Kanal oder besser durch mehrere Kanäle zu einem elastischen Kopf der Vorrichtung transportiert. Der elastische Kopf stellt dabei erfindungsgemäß sicher, dass sich die Form des Kopfs zumindest geringfügig an die äußeren Konturen des zu beschichtenden Objekts anpasst. Vor allem diese überraschend gefundene Erkenntnis ermöglicht eine einfache Handhabung der erfindungsgemäßen Vorrichtung.

Eine insbesondere vom hygienischen Standpunkt aus besonders interessante Ausgestaltung der Erfindung beruht auf der zusätzlichen erfindungsgemäßen Idee, den elastischen Kopf als eine sich drehende Walze oder als einen in alle Richtungen frei drehbare Kugel auszugestalten. Wenn an der Oberfläche der Walze oder der Kugel eine ausreichende Rauigkeit und/oder eine ausreichende Benetzbarkeit mit der Flüssigkeit gegeben ist, fördert der sich drehende elastische Kopf die Flüssigkeit aus dem Inneren der Vorrichtung zu der zu beschichtenden Oberfläche. Ohne über die Oberfläche des zu beschichtenden medizinischen Implantats reiben zu müssen, wird dann der Flüssigkeitsfilm nach Art eines Deo-Rollers aufgetragen. Die Elastizität des drehbar gelagerten elastischen Kopfs sollte dazu so bemessen sein, dass sich die Verformung nicht bis zur Lagerung des elastischen Kopfs im Behälter fortpflanzt oder wenn, dann nur so wenig, dass die Drehbarkeit des elastischen Kopfs in der Vorrichtung möglichst wenig beeinträchtigt wird. Dazu kann der Behälter einen elastischen Bereich im Bereich der Lagerung umfassen.

Für einen initialen antibiotischen Schutz reicht es aus, wenn für einen Zeitraum von 24 bis 72 Stunden hinreichend hohe Antibiotikum- oder Antibiotika-Konzentrationen an den Implantatoberflächen vorhanden sind. Daher kann auch bei der lokalen Einbringung von einfachen in Wasser löslichen Antibiotika in eine Flüssigkeit ein ausreichender temporärer, lokaler, antibiotischer Schutz des medizinischen Implantats erzielt werden.

Anstatt also das medizinische Implantat lange im Voraus bei der Herstellung zu beschichten, kann es auch unmittelbar vor dem Einsetzen beschichtet werden. Dadurch können auch relativ kurzlebige Beschichtungen verwendet werden. Zudem kann sogar eine noch flüssige Schicht verwendet werden, was neue Anwendungsgebiete eröffnet und neue Wirkstoffe zugänglich macht.

Eine erfindungsgemäße Vorrichtung ist so gestaltet, dass kein Sprühnebel das OP-Feld kontaminieren kann und dass auch Bürsten, Pinsel oder andere Haare oder Borsten oder sonstige Partikel freisetzende Vorrichtungsbestandteile vermieden werden.

Im Folgenden werden Ausführungsbeispiele der Erfindung anhand von zwei schematisch dargestellten Figuren erläutert, ohne jedoch dabei die Erfindung zu beschränken. Dabei zeigt:
Figur 1: eine schematische Querschnittansicht einer erfindungsgemäßen Vorrichtung und
Figur 2: eine schematische Querschnittansicht einer zweiten erfindungsgemäßen Vorrichtung.

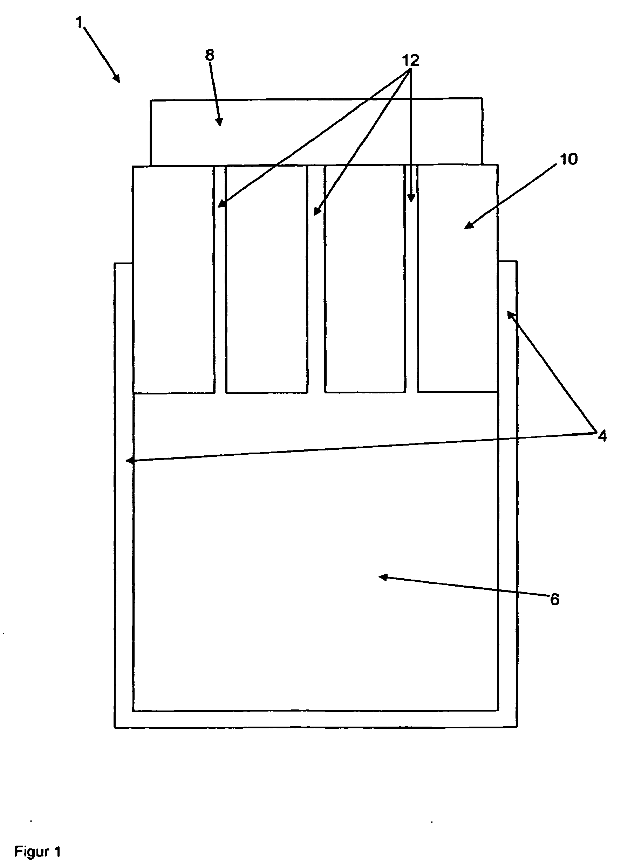

Figur 1 zeigt eine schematische Querschnittansicht einer erfindungsgemäßen Vorrichtung 1. Die Vorrichtung 1 umfasst einen Behälter 4 in Form eines oben offenen Topfs. Die Seitenwände des Behälters 4 sind zylindrisch und von gleichmäßiger Dicke.

Im Inneren des Behälters 4 befindet sich eine wässrige Flüssigkeit 6, die eine pharmazeutisch aktive Substanz beinhaltet. Die Flüssigkeit 6 ist beispielsweise eine wässrige Lösung mit Antibiotika, mit der ein medizinisches Implantat (nicht gezeigt) beschichtet werden soll.

In der Öffnung des Behälters 4 ist ein elastischer Kopf 8 in Form eines Schwamms 8 auf einem Pfropfen 10 angeordnet. Der Pfropfen 10 steckt in der Öffnung des Behälters 4 und verschließt ihn auf allen Seiten. Im Pfropfen 10 sind mehrere Kanäle 12 angeordnet, durch die die Flüssigkeit 6 aus dem Inneren des Behälters 4 bis zum porösen Schwamm 8 geleitet wird. Der Schwamm 8 ist hydrophil und somit zur Aufnahme der Flüssigkeit 6 gut geeignet. So kann sich der Schwamm 8 durch die Kanäle 12 mit der Flüssigkeit 6 vollsaugen.

Der Pfropfen 10 und die Innenwände des Behälters 4 können aus einem hydrophoben Material bestehen oder mit einem hydrophoben Material beschichtet sein. Der Pfropfen 10 kann in der Öffnung des Behälters 4 verschoben werden, ist also linear in der Öffnung des Behälters beweglich und wird durch die Innenwände des Behälters 4 geführt.

Mit der gezeigten Vorrichtung 1 kann ein erfindungsgemäßes Verfahren durchgeführt werden. Die Vorrichtung 1 wird dazu mit dem elastischen Kopf 8 nach unten über eine zu beschichtende Oberfläche eines medizinischen Implantats gestrichen. Durch den Druck, der auf den Schwamm 8 ausgeübt wird, wird die in dem Schwamm 8 enthaltene Flüssigkeit 6 ausgepresst und als Flüssigkeitsfilm auf die Oberfläche des medizinischen Implantats übertragen. Durch den manuell ausgeübten Druck wird gleichzeitig die Flüssigkeit 6 aus dem Inneren des Behälters 4 in den Schwamm 8 gedrückt, so dass der Flüssigkeitsnachschub an die Oberfläche des elastischen Kopfs 8 sichergestellt ist. Der Flüssigkeitsfilm trocknet schnell auf der Oberfläche des medizinischen Implantats und hinterlässt eine Schicht einer pharmazeutisch wirksamen Substanz, beispielsweise ein Antibiotikum oder ein Antibiotika-Gemisch. Das beschichtete medizinische Implantat ist dann bereit für eine OP, das heißt bereit zum Einsetzen.

Die Zusammensetzung der Flüssigkeit 6 kann dabei kurz vor der Operation bestimmt und hergestellt werden, indem durch die Wände und/oder den Boden des Behälters 4 mit einer Spritze geeignete pharmazeutisch wirksame Substanzen zugegeben werden. Alternativ kann der Behälter 4 auch einfach vor der Anwendung mit einer geeigneten Flüssigkeit 8 befüllt werden, die durch die Öffnung des Behälters 4 eingefüllt wird, bevor der Pfropfen 10 in die Öffnung gesteckt wird. Dazu können der Behälter 4 und der Pfropfen 10 mit dem elastischen Kopf 8 zuvor in einer sterilen Verpackung gelagert sein, die auch mit einem sterilisierenden Gas gefüllt sein kann. Auch der Behälter 4 selbst kann mit einem solchen sterilisierenden Gas gefüllt sein.

Die Beschichtungsvorrichtung 1 ist aus Polypropylen gefertigt, hat eine Höhe von ca. 20 cm und einen Durchmesser von 8 cm. Der Pfropfen 10 besteht ebenfalls aus Polypropylen und ist im Presssitz in den oberen Bereich des Innenraums des Behälters 4 eingesetzt. Der Behälter 4 kann vor der Anwendung zusätzlich durch eine Aluminiumverbundfolie (nicht gezeigt) keimdicht verschlossen sein, die die Öffnung des Behälters 4 verschließt.

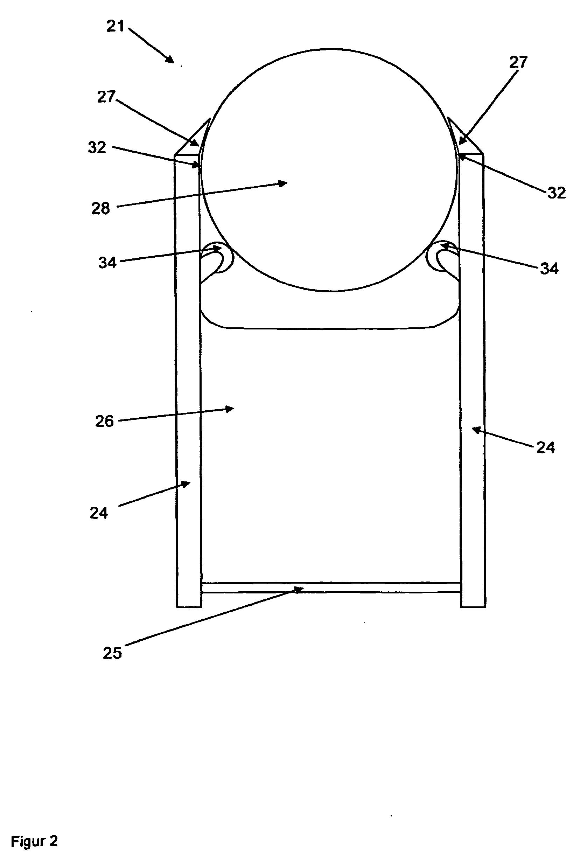

Figur 2 zeigt eine schematische Querschnittansicht einer zweiten erfindungsgemäßen Vorrichtung 21, die für ein erfindungsgemäßes Verfahren geeignet ist. Die Vorrichtung 21 umfasst einen Behälter 24 mit einer bodenseitigen, durchstechbaren, elastischen Membran 25, die den Behälter 24 auf der Unterseite vollständig abschließt. Im Inneren des Behälters 24 befindet sich eine Flüssigkeit 26 die eine pharmazeutisch aktive Substanz enthält, mit der das medizinische Implantat beschichtet ist, wenn das Lösungsmittel der Flüssigkeit, in der die pharmazeutisch aktive Substanz gelöst ist, verdampft ist. Durch die Membran 25 kann mit Spritzen gestochen werden, um den Behälter 24 mit der Flüssigkeit 26 zu füllen oder um die im Behälter 24 enthaltene Flüssigkeit 26 mit der pharmazeutisch aktiven Substanz zu versetzen.

An der Oberseite des Behälters 24 ist an den Behälterwänden eine umlaufende Lippe 27 aus einem elastischen Material angeordnet. Die Oberseite des Behälters 24 ist durch eine Kugel 28 als elastischer Kopf 28 der Vorrichtung 21 verschlossen. Die Kugel 28 ist aus einem elastischen biokompatiblen Polymer gefertigt und hat eine Oberflächenrauigkeit von 10 µm. Zwischen der Kugel 28, den Wänden des Behälters 24 und der Lippe 27 ist ein Spalt 32, der als Kanal 32 für die Flüssigkeit 26 im Inneren des Behälters 24 dient.

Die Kugel 28 ist in dem Behälter 24 durch die Lippe 27 und durch ein Kugellager 34 gehaltert. Dadurch ist die Kugel 28 frei beweglich, also in alle Richtungen drehbar. Das Kugellager 34 ist für die Ausführung der Erfindung nicht notwendig, stattdessen kann die Kugel 28 auch einfach auf einem Flüssigkeitsfilm der Flüssigkeit 26 in einer Halterung am oberen Ende des Behälters 24 gleitend gelagert sein.

Wenn die Vorrichtung 21 mit der Kugel 28 nach unten über ein medizinisches Implantat (nicht gezeigt) gerollt wird, fördert sie die Flüssigkeit an ihrer rauen Oberfläche aus dem Inneren der Vorrichtung 21 durch den Spalt 32 an die äußere Oberfläche der Vorrichtung 21, die durch die Kugel 28 gebildet wird. Wenn die Kugel 28 durch den Druck, den das Gewicht der Vorrichtung 21 und gegebenenfalls der Benutzer der Vorrichtung 21 ausübt, in das Kugellager 34 gepresst wird, hat der Spalt 32 eine Breite von ungefähr 50 µm. Die Spaltbreite kann aber auch auf die Konsistenz der Flüssigkeit 26 und/oder auf die Rauigkeit der Kugel 28 abgestimmt sein. Je zähflüssiger die Flüssigkeit 26 ist, desto breiter wird der Spalt 32 gewählt.

Wenn kein Kugellager 34 vorgesehen ist, können statt dem Spalt 32 Kanäle als flache Rillen in der Lagerung des Behälters angeordnet sein, die eine Breite von ungefähr 1 bis 10 mm und eine Tiefe von 50 bis 200 µm haben. In diesen Kanälen kann dann die Flüssigkeit 26 aus der Vorrichtung 21 heraustransportiert werden. Für die erfindungsgemäße Ausführungsform nach Figur 2 ist das Besondere und Erfindungsgemäße darin zu sehen, dass die Flüssigkeit 26 eine pharmazeutisch aktive Substanz enthält,
mit der ein medizinisches Implantat beschichtet werden soll, und das gleichzeitig die Kugel 28 den elastischen Kopf 28 der Vorrichtung 21 bildet, wobei durch die Drehung der Kugel 28 die Flüssigkeit 26 aus dem Inneren der Vorrichtung 21 gefördert wird und aufgrund der Elastizität der Kugel 28 die Flüssigkeit 26 flächig auch auf unebenen Konturen des medizinischen Implantats aufgetragen wird.

Erfindungsgemäß können übliche Zweymüller-Hüftendoprothesen kurz mit den mit Flüssigkeit befüllten Vorrichtungen 1, 21 überstrichen werden. Die Zweymüller-Hüftprothesen haben dann einen Film einer Flüssigkeit 6, 26 an der Schaftoberfläche. Nach dem Austrocknen des Flüssigkeitsfilms können die Zweymüller-Hüftprothesen dann einen weißen Belag an der Schaftoberfläche haben, in dem sich die pharmazeutisch aktive Substanz befindet. Die Hüftprothesen sind bereit zum Einsatz bei einer Operation.

Nach der Beschichtung mit der Flüssigkeit 6, 26 kann das noch feuchte medizinische Implantat auch noch mit einem Pulver beschichtet werden. Das Pulver enthält eine zweite pharmazeutisch aktive Substanz, vorzugsweise eine das Knochenwachstum fördernde Substanz, wie Calciumphosphat. Durch den Flüssigkeitsfilm auf dem medizinischen Implantat haftet das Pulver gut auf dessen Oberfläche. Daraus resultiert eine Flüssigkeits-Pulver-Beschichtung auf der zu beschichtenden Oberfläche des medizinischen Implantats.

Im Folgenden werden Beispiele zur Herstellung von Flüssigkeiten für ein erfindungsgemäßes Verfahren und ein weiteres Beispiel für eine erfindungsgemäße Vorrichtung ausgeführt.

Es werden 16,0 g Gentamicinsulfat (Fujian Fukang Ltd.) mit 4,0 ml pyrogenfreies steriles Wasser bei Raumtemperatur vermischt. Nach 24 Stunden Rühren bei Raumtemperatur mit einem Magnetrührer hat sich eine ölig-viskose gelbliche Lösung gebildet. Es ergibt sich eine Beschichtungslösung mit Gentamicinsulfat als Flüssigkeit zur Beschichtung eines medizinischen Implantats.

Herstellung einer Beschichtungslösung mit der Zweifachkombination Gentamicinsulfat und Clindamycinhydrochlorid:
Es werden 12,0 g Gentamicinsulfat (Fujian Fukang Ltd.), 4,0 g Clindamycinhydrochlorid (Sigma-Aaldrich) mit 4,0 ml pyrogenfreies steriles Wasser bei Raumtemperatur vermischt. Nach 24 Stunden Rühren bei Raumtemperatur mit einem Magnetrührer hat sich eine ölig-viskose gelbliche Lösung gebildet.

Herstellung einer Beschichtungslösung mit der Dreifachkombination Gentamicinsulfat, Clindamycinhydrochlorid und Vancomycinhydrochlorid:
Es werden 4,0 g Gentamicinsulfat (Fujian Fukang Ltd.), 4,0 g Clindamycinhydrochlorid (Sigma-Aaldrich) und 4,0 g Vancomycinhydrochlorid (Sigma-Aldrich) mit 8,0 ml pyrogenfreies steriles Wasser bei Raumtemperatur vermischt. Nach 24 Stunden Rühren bei Raumtemperatur mit einem Magnetrührer hat sich eine viskose gelbliche Lösung gebildet.

Herstellung einer Beschichtungslösung mit der Zweifachkombination Gentamicinsulfat und Clindamycinhydrochlorid:
Es werden 3,0 g Gentamicinsulfat (Fujian Fukang Ltd.), 1,0 g Clindamycinhydrochliorid (Sigma-Aaldrich) mit 1,0 ml pyrogenfreies steriles Wasser bei Raumtemperatur vermischt. Nach 24 Stunden Rühren bei Raumtemperatur mit einem Magnetrührer hat sich eine ölig-viskose gelbliche Lösung gebildet.

Herstellung einer Beschichtungslösung mit der Dreifachkombination Gentamicinsulfat, Clindamycinhydrochlorid und Vancomycinhydrochlorid:
Es werden 2,0 g Gentamicinsulfat (Fujian Fukang Ltd.), 1,0 g Clindamycinhydrochlorid (Sigma-Aaldrich) und 1,0 g Vancomycinhydrochlorid (Sigma-Aldrich) mit 1,0 ml pyrogenfreies steriles Wasser bei Raumtemperatur vermischt. Nach 24 Stunden Rühren bei Raumtemperatur mit einem Magnetrührer hat sich eine viskose gelbliche Lösung gebildet.

Mit konventionellen 10 ml Kunststoffspritzen werden jeweils die Beschichtungslösungen der Beispiele 2-4 aufgezogen und dann nach Aufsetzen einer Kanüle durch die Bodenplatte der in den Behälter einer erfindungsgemäßen Vorrichtung gespritzt.

Die so befüllten Vorrichtungen werden auf übliche Zweymüller-Hüftprothesen gerollt. Es bildet sich dabei ein viskoser Film auf den Implantatoberflächen aus.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 21: Vorrichtung
- 4, 24: Behälter
- 6, 26: Flüssigkeit
- 8: elastischer Kopf / Schwamm
- 10: Pfropfen
- 12, 32: Kanal / Spalt
- 25: Membran
- 28: elastischer Kopf / Kugel
- 34: Kugellager

## Patentansprüche

1. Vorrichtung (1, 21) zum Beschichten eines medizinischen Implantats, wobei die Vorrichtung (1, 21) einen Behälter (4, 24) umfasst, der eine Flüssigkeit (6, 26) beinhaltet, wobei die Flüssigkeit (6, 26) zumindest eine pharmazeutisch aktive Substanz umfasst, die Vorrichtung (1, 21) einen elastischen Kopf (8, 28) zum Auftragen der Flüssigkeit (6, 26) auf einer zu beschichtenden Oberfläche umfasst, der über zumindest einen Kanal (12, 32) mit dem Inneren des Behälters (4, 24) verbunden ist, so dass bei einem Überstreichen oder Überrollen der zu beschichtenden Oberfläche mit dem elastischen Kopf (8, 28) die Flüssigkeit (6, 26) die zu beschichtende Oberfläche benetzt, **dadurch gekennzeichnet, dass**
der Boden und/oder zumindest eine Wand des Behälters (4, 24) zumindest bereichsweise eine elastische durchstechbare Membran (25) umfasst.

2. Vorrichtung (1, 21) nach Anspruch 1, **dadurch gekennzeichnet, dass** die Membran (25) aus einem biokompatiblen Elastomer gebildet ist.

3. Vorrichtung (1, 21) nach Anspruch 1 oder 2, **dadurch gekennzeichnet, dass** die Membran (25) durch einen Deckel abdeckbar und/oder abdichtbar ist.

4. Vorrichtung (1, 21) nach einem der vorangehenden Ansprüche, **dadurch gekennzeichnet, dass**
der Behälter (4, 24) elastische Wände hat, so dass bei einem manuellen Druck auf die elastischen Behälterwände die Flüssigkeit (6, 26) durch den zumindest einen Kanal (12, 32) zur Oberfläche des elastischen Kopfs (8, 28) strömt.

5. Vorrichtung (1, 21) nach einem der vorangehenden Ansprüche, **dadurch gekennzeichnet, dass**
der elastische Kopf (8, 28) eine rotationssymmetrische Walze (28) ist, die um ihre Symmetrieachse drehbar an einem Ende des Behälters (4, 24) gelagert ist.

6. Vorrichtung (1, 21) nach einem der vorangehenden Ansprüche, **dadurch gekennzeichnet, dass**
der elastische Kopf (8, 28) eine Kugel (28) ist, die um zumindest eine Achse drehbar an einem Ende des Behälters (4, 24) gelagert ist.

7. Vorrichtung (1, 21) nach einem der Ansprüche 5 oder 6, **dadurch gekennzeichnet, dass** zumindest ein Kanal (12, 32) zwischen der Behälterwand und dem elastischen Kopf (8, 28) gebildet ist.

8. Vorrichtung (1, 21) nach einem der vorangehenden Ansprüche, **dadurch gekennzeichnet, dass**
der elastische Kopf (8, 28) einen porösen elastischen Schwamm (8) umfasst.

9. Vorrichtung (1, 21) nach einem der vorangehenden Ansprüche, **dadurch gekennzeichnet, dass**
der elastische Kopf (8, 28) linear verschiebbar im Behälter (4, 24) angeordnet ist.

10. Vorrichtung (1, 21) nach einem der vorangehenden Ansprüche, **dadurch gekennzeichnet, dass**
zumindest ein Kanal (12, 32) im elastischen Kopf (8, 28) ausgebildet ist, vorzugsweise durch Poren eines Schwamms (8).

11. Vorrichtung (1, 21) nach einem der vorangehenden Ansprüche, **dadurch gekennzeichnet, dass**
am elastischen Kopf (8, 28) ein unelastischer Pfropfen (10) angeordnet ist, der den Behälter (4, 24) einseitig abschließt und der zumindest einen Kanal (12, 32) umfasst und/oder zumindest einen Kanal (12, 32) zwischen dem Pfropfen (10) und dem Behälter (4, 24) bildet, wobei der Pfropfen (10) vorzugsweise linear verschiebbar im Behälter (4, 24) angeordnet ist.

12. Vorrichtung (1, 21) nach einem der vorangehenden Ansprüche, **dadurch gekennzeichnet, dass**
der elastische Kopf (8, 28) eine raue Oberfläche hat, vorzugsweise eine Rauigkeit von 0,5 µm bis 100 µm hat, besonders bevorzugt 1 µm bis 10 µm, ganz besonders bevorzugt 2 µm.

13. Vorrichtung (1, 21) nach einem der vorangehenden Ansprüche, **dadurch gekennzeichnet, dass**
der elastische Kopf (8, 28) hydrophil ist und die Flüssigkeit (6, 26) eine wässrige Lösung ist.

14. Vorrichtung (1, 21) nach einem der vorangehenden Ansprüche, **dadurch gekennzeichnet, dass**
der elastische Kopf (8, 28) den Behälter (4, 24) bis auf die Verbindung durch den zumindest einen Kanal (12, 32) nach außen abschließt.

15. Vorrichtung (1, 21) nach einem der vorangehenden Ansprüche, **dadurch gekennzeichnet, dass**
die Vorrichtung (1, 21) eine Lagerung (34) für den elastischen Kopf (8, 28) umfasst, die mit dem Behälter (4, 24) verbunden ist oder mit dem Behälter (4, 24) in einem ausgeformt ist.

16. Vorrichtung (1, 21) nach einem der vorangehenden Ansprüche, **dadurch gekennzeichnet, dass**
der elastische Kopf (8, 28) einen Elastizitätsmodul von weniger als 2000 MPa, bevorzugt von weniger als 500 MPa, besonders bevorzugt zwischen 1 und 100 MPa hat.

17. Vorrichtung (1, 21) nach einem der vorangehenden Ansprüche, **dadurch gekennzeichnet, dass**
zumindest ein Kanal (12, 32), bevorzugt alle Kanäle (12, 32), einen Querschnitt von unter 500 µm hat, vorzugsweise mit einem Querschnitt von unter 200 µm.

18. Vorrichtung (1, 21) nach einem der vorangehenden Ansprüche, **dadurch gekennzeichnet, dass**
zumindest ein Kanal (12, 32) ein Spalt (32) mit einer Breite von unter 500 µm ist, vorzugsweise mit einer Breite von unter 200 µm, bevorzugt alle Kanäle (12, 32) solche Spalten (32) sind.

19. Vorrichtung (1, 21) nach einem der vorangehenden Ansprüche, **dadurch gekennzeichnet, dass**
der elastische Kopf (8, 28) und/oder der Behälter (4, 24) ein biokompatibles Material umfasst oder umfassen oder aus einem biokompatiblen Material gefertigt ist oder sind, vorzugsweise zumindest die Oberfläche des elastischen Kopfs (8, 28) aus einem biokompatiblen Elastomer oder Elastomergemisch besteht.

20. Vorrichtung (1, 21) nach einem der vorangehenden Ansprüche, **dadurch gekennzeichnet, dass**
das durch den Behälter (4, 24) und den elastischen Kopf (8, 28) eingeschlossene Volumen zwischen 0,5 ml und 1000 ml, vorzugsweise zwischen 1 ml und 100 ml beträgt.

21. Vorrichtung (1, 21) nach einem der vorangehenden Ansprüche, **dadurch gekennzeichnet, dass**
der Behälter (4, 24) sterile Luft oder ein steriles Gas beinhaltet.

22. Vorrichtung (1, 21) nach einem der vorangehenden Ansprüche, **dadurch gekennzeichnet, dass**
zwischen dem elastischen Kopf (8, 28) und dem Behälter (4, 24) wenigstens eine offenporige poröse Schicht angeordnet ist, vorzugsweise im Inneren der Vorrichtung (1, 21).

23. Vorrichtung (1, 21) nach einem der vorangehenden Ansprüche, **dadurch gekennzeichnet, dass**
die Flüssigkeit (6, 26) eine wässrige Gentamicinsulfat-Lösung umfasst, vorzugsweise umfassend Stabilisatoren, wobei besonders eine wässrige Gentamicinsulfat-Lösung mit einem Gentamicinsulfat-Gehalt von 0,5 bis 88 Gewichtsprozent bevorzugt ist.

24. Vorrichtung (1, 21) nach Anspruch 23, **dadurch gekennzeichnet, dass** die Flüssigkeit (6, 26) eine Gentamicinsulfat-Lösung mit einem Gentamicinsulfat-Gehalt von 10 bis 88 Gewichtsprozent ist, besonders bevorzugt eine Gentamicinsulfat-Lösung mit einem Gentamicinsulfat-Gehalt von 80 Gewichtsprozent ist.

25. Vorrichtung (1, 21) nach einem der vorangehenden Ansprüche, **dadurch gekennzeichnet, dass**
die Kanäle (12, 32) als Zwischenräume von Fasern ausgebildet sind, wobei die Fasern den elastischen Kopf (8, 28) bilden.

## Claims

1. Device (1, 21) for coating a medical implant, whereby the device (1, 21) comprises a container (4, 24) that contains a liquid (6, 26), whereby the liquid (6, 26) comprises at least one pharmaceutically active substance, whereby the device (1, 21) comprises an elastic head (8, 28) for application of the liquid (6, 26) onto a surface to be coated that is connected to the inside of the container (4, 24) through at least one channel (12, 32) such that the liquid (6, 26) wets the surface to be coated when the elastic head (8, 28) passes or rolls over the surface to be coated, **characterised in that**
at least part of the floor and/or at least one wall of the container (4, 24) comprises an elastic membrane (25) that can be punctured.

2. Device (1, 21) according to claim 1, **characterised in that** the membrane (25) is made from a biocompatible elastomer.

3. Device (1, 21) according to claim 1, **characterised in that** the membrane (25) can be covered and/or is sealed by means of a lid.

4. Device (1, 21) according to any one of the preceding claims, **characterised in that**
the container (4, 24) has elastic walls such that the liquid (6, 26) flows through the at least one channel (12, 32) to the surface of the elastic head (8, 28) upon a manual pressure being applied to the elastic container walls.

5. Device (1, 21) according to any one of the preceding claims, **characterised in that**
the elastic head (8, 28) is a rotationally symmetrical roller (28) that is supported as by a bearing at one end of the container (4, 24) such that the roller can rotate about its symmetry axis.

6. Device (1, 21) according to any one of the preceding claims, **characterised in that**
the elastic head (8, 28) is a sphere (28) that is supported as by a bearing at one end of the container (4, 24) such it can rotate about at least one axis.

7. Device (1, 21) according to any one of the claims 5 or 6, **characterised in that** at least one channel (12, 32) is formed between the container wall and the elastic head (8, 28).

8. Device (1, 21) according to any one of the preceding claims, **characterised in that**
the elastic head (8, 28) comprises a porous elastic sponge (8).

9. Device (1, 21) according to any one of the preceding claims, **characterised in that**
the elastic head (8, 28) is arranged in the container (4, 24) in a manner such that it can be shifted linearly.

10. Device (1, 21) according to any one of the preceding claims, **characterised in that**
at least one channel (12, 32) is formed in the elastic head (8, 28), preferably through pores of a sponge (8).

11. Device (1, 21) according to any one of the preceding claims, **characterised in that**
an inelastic plug (10) is arranged on the elastic head (8, 28) and closes off the container (4, 24) on one side and comprises at least one channel (12, 32) and/or at least forms one channel (12, 32) between the plug (10) and the container (4, 24), whereby the plug (10) preferably is arranged in the container (4, 24) in a manner such that it can be shifted linearly.

12. Device (1, 21) according to any one of the preceding claims, **characterised in that**
the elastic head (8, 28) has a rough surface, preferably has a roughness of 0.5 µm to 100 µm, particularly preferably of 1 µm to 10 µm, more particularly preferably of 2 µm.

13. Device (1, 21) according to any one of the preceding claims, **characterised in that**
the elastic head (8, 28) is hydrophilic and the liquid (6, 26) is an aqueous solution.

14. Device (1, 21) according to any one of the preceding claims, **characterised in that**
the elastic head (8, 28) closes the container (4, 24) with respect to the outside except for the connection through the at least one channel (12, 32).

15. Device (1, 21) according to any one of the preceding claims, **characterised in that**
the device (1, 21) comprises a bearing (34) for the elastic head (8, 28) that is connected to the container (4, 24) or is provided to be the same part as the container (4, 24).

16. Device (1, 21) according to any one of the preceding claims, **characterised in that**
the elastic head (8, 28) has a modulus of elasticity of less than 2,000 MPa, preferably less than 500 MPa, particularly preferably between 1 and 100 MPa.

17. Device (1, 21) according to any one of the preceding claims, **characterised in that**
at least one channel (12, 32), preferably all channels (12, 32), has/have a cross-section of less than 500 µm, preferably a cross-section of less than 200 µm.

18. Device (1, 21) according to any one of the preceding claims, **characterised in that**
at least one channel (12, 32) is a gap (32) with a width of less than 500 µm, preferably with a width of less than 200 µm, preferably all channels (12, 32) are said gaps (32).

19. Device (1, 21) according to any one of the preceding claims, **characterised in that**
the elastic head (8, 28) and/or the container (4, 24) comprise(s) a biocompatible material or is/are made of a biocompatible material, preferably at least the surface of the elastic head (8, 28) consists of a biocompatible elastomer or elastomer mixture.

20. Device (1, 21) according to any one of the preceding claims, **characterised in that**
the volume enclosed by the container (4, 24) and the elastic head (8, 28) is between 0.5 ml and 1,000 ml, preferably between 1 ml and 100 ml.

21. Device (1, 21) according to any one of the preceding claims, **characterised in that**
the container (4, 24) contains sterile air or a sterile gas.

22. Device (1, 21) according to any one of the preceding claims, **characterised in that**
at least one open-pore porous layer is arranged between the elastic head (8, 28) and the container (4, 24), preferably on the inside of the device (1, 21).

23. Device (1, 21) according to any one of the preceding claims, **characterised in that**
the liquid (6, 26) comprises an aqueous gentamicin sulfate solution, preferably comprising stabilisers, whereby, in particular, an aqueous gentamicin sulfate solution with a gentamicin sulfate content of 0.5 to 88 % by weight is preferred.

24. Device (1, 21) according to claim 23, **characterised in that** the liquid (6, 26) is a gentamicin sulfate solution with a gentamicin sulfate content of 10 to 88 % by weight, particularly preferably a gentamicin sulfate solution with a gentamicin sulfate content of 80 % by weight.

25. Device (1, 21) according to any one of the preceding claims, **characterised in that**
the channels (12, 32) are provided as intervening spaces of fibres, whereby the fibres form the elastic head (8, 28).

## Revendications

1. Dispositif (1, 21) de revêtement d'un implant médical, le dispositif (1, 21) comprenant un réservoir (4, 24), qui contient un liquide (6, 26), le liquide (6, 26) comprenant au moins une substance pharmaceutiquement active, le dispositif (1, 21) comprenant une tête élastique (8, 28) destinée à appliquer le liquide (6, 26) sur une surface à revêtir, qui est reliée à l'intérieur du réservoir (4, 24) par le biais d'au moins un canal (12, 32), de telle sorte que, lors d'un balayage ou d'un roulement sur la surface à revêtir au moyen de la tête élastique (8, 28), le liquide (6, 26) mouille la surface à revêtir, **caractérisé en ce que** le fond et/ou au moins une paroi du réservoir (4, 24) comprend/comprennent au moins sur certaines parties une membrane élastique (25) perforable.

2. Dispositif (1, 21) selon la revendication 1, **caractérisé en ce que** la membrane (25) est formée en un élastomère biocompatible.

3. Dispositif (1, 21) selon la revendication 1 ou 2, **caractérisé en ce que** la membrane (25) peut être recouverte et/ou fermée hermétiquement par un couvercle.

4. Dispositif (1, 21) selon l'une des revendications précédentes, **caractérisé en ce que**
le réservoir (4, 24) comporte des parois élastiques, de telle sorte que, lors d'une pression manuelle sur les parois élastiques du réservoir, le liquide (6, 26) s'écoule jusqu'à la surface de la tête élastique (8, 28) par l'au moins un canal (12, 32).

5. Dispositif (1, 21) selon l'une des revendications précédentes, **caractérisé en ce que**
la tête élastique (8, 28) est un rouleau à symétrie de révolution (28), qui est monté sur une extrémité du réservoir (4, 24) de manière rotative sur son axe de symétrie.

6. Dispositif (1, 21) selon l'une des revendications précédentes, **caractérisé en ce que**
la tête élastique (8, 28) est une bille (28), qui est montée sur une extrémité du réservoir (4, 24) de manière rotative sur au moins un axe.

7. Dispositif (1, 21) selon la revendication 5 ou 6, **caractérisé en ce que** au moins un canal (12, 32) est formé entre la paroi de réservoir et la tête élastique (8, 28).

8. Dispositif (1, 21) selon l'une des revendications précédentes, **caractérisé en ce que**
la tête élastique (8, 28) comprend une éponge (8) élastique poreuse.

9. Dispositif (1, 21) selon l'une des revendications précédentes, **caractérisé en ce que**
la tête élastique (8, 28) est disposée mobile de manière linéaire dans le réservoir (4, 24).

10. Dispositif (1, 21) selon l'une des revendications précédentes, **caractérisé en ce que**
au moins un canal (12, 32) est formé dans la tête élastique (8, 28), de préférence à travers des pores d'une éponge (8).

11. Dispositif (1, 21) selon l'une des revendications précédentes, **caractérisé en ce que**
un bouchon non élastique (10), qui ferme le réservoir (4, 24) d'un côté et qui comprend au moins un canal (12, 32) et/ou qui forme au moins un canal (12, 32) entre le bouchon (10) et le réservoir (4, 24), est disposé au niveau de la tête élastique (8, 18), le bouchon (10) étant disposé de préférence mobile de manière linéaire dans le réservoir (4, 24).

12. Dispositif (1, 21) selon l'une des revendications précédentes, **caractérisé en ce que**
la tête élastique (8, 28) comporte une surface rugueuse, présente de préférence une rugosité de 0,5 µm à 100 µm, de manière particulièrement préférentielle de 1 µm à 10 µm, idéalement de 2 µm.

13. Dispositif (1, 21) selon l'une des revendications précédentes, **caractérisé en ce que**
la tête élastique (8, 28) est hydrophile et le liquide (6, 26) est une solution aqueuse.

14. Dispositif (1, 21) selon l'une des revendications précédentes, **caractérisé en ce que**
la tête élastique (8, 28) isole le réservoir (4, 24) de l'extérieur à l'exception de la liaison par l'au moins un canal (12, 32).

15. Dispositif (1, 21) selon l'une des revendications précédentes, **caractérisé en ce que**
le dispositif (1, 21) comprend un palier (34) pour la tête élastique (8, 28), qui est relié au réservoir (4, 24) ou qui est formé d'un seul tenant avec le réservoir (4,24).

16. Dispositif (1, 21) selon l'une des revendications précédentes, **caractérisé en ce que**
la tête élastique (8, 28) comporte un module d'élasticité inférieur à 2000 MPa, de préférence inférieur à 500 MPa, de manière particulièrement préférentielle compris entre 1 et 100 MPa.

17. Dispositif (1, 21) selon l'une des revendications précédentes, **caractérisé en ce que**
au moins un canal (12, 32), de préférence tous les canaux (12, 32), présente une section transversale inférieure à 500 µm_{[JT1]}, de préférence avec une section transversale inférieure à 200 µm.

18. Dispositif (1, 21) selon l'une des revendications précédentes, **caractérisé en ce que**
au moins un canal (12, 32) est un interstice (32) ayant une largeur inférieure à 500 µm, de préférence une largeur inférieure à 200 µm, tous les canaux (12, 32) sont de préférence des interstices (32) de ce type.

19. Dispositif (1, 21) selon l'une des revendications précédentes, **caractérisé en ce que**
la tête élastique (8, 28) et/ou le réservoir (4, 24) comprend ou comprennent un matériau biocompatible ou est ou sont fabriqué(e)(s) en un matériau biocompatible, au moins la surface de la tête élastique (8, 28) est constituée de préférence d'un élastomère ou d'un mélange élastomère biocompatible.

20. Dispositif (1, 21) selon l'une des revendications précédentes, **caractérisé en ce que**
le volume renfermé par le réservoir (4, 24) et la tête élastique (8, 28) est compris entre 0,5 ml et 1000 ml, de préférence entre 1 ml et 100 ml.

21. Dispositif (1, 21) selon l'une des revendications précédentes, **caractérisé en ce que**
le réservoir (4, 24) contient de l'air stérile ou un gaz stérile.

22. Dispositif (1, 21) selon l'une des revendications précédentes, **caractérisé en ce que**
au moins une couche poreuse à pores ouvertes est disposée entre la tête élastique (8, 28) et le réservoir (4, 24), de préférence à l'intérieur du dispositif (1,21).

23. Dispositif (1, 21) selon l'une des revendications précédentes, **caractérisé en ce que**
le liquide (6, 26) comprend une solution de sulfate de gentamicine aqueuse, comprenant de préférence des stabilisants, une solution de sulfate de gentamicine aqueuse avec une teneur en sulfate de gentamicine de 0,5 à 88 pour cent en poids étant particulièrement préférentielle.

24. Dispositif (1, 21) selon la revendication 23, **caractérisé en ce que** le liquide (6, 26) est une solution de sulfate de gentamicine avec une teneur en sulfate de gentamicine de 10 à 88 pour cent en poids, de manière particulièrement préférentielle une solution de sulfate de gentamicine avec une teneur en sulfate de gentamicine de 80 pour cent en poids.

25. Dispositif (1, 21) selon l'une des revendications précédentes, **caractérisé en ce que**
les canaux (12, 32) sont réalisés sous la forme d'espaces intermédiaires de fibres, les fibres formant la tête élastique (8, 28).
